# EUROPEAN PATENT SPECIFICATION

(11) **EP 4 430 448 B1**
(45) Date of publication and mention of the grant of the patent: **21.10.2026**
(21) Application number: 22893583.9
(22) Date of filing: 10.11.2022
(51) Int. Cl.: G02C 7/10, G02C 7/12, G02F 1/01, G02F 1/13, G02F 1/137, G02F 1/17, G02F 1/133, G02F 1/1347, G02F 1/31

(54) **LIGHT PROTECTION SYSTEM AND METHOD**
LICHTSCHUTZSYSTEM UND -VERFAHREN
SYSTÈME ET PROCÉDÉ DE PROTECTION CONTRE LA LUMIÈRE

(30) Priority: 11.11.2021 US 202163263923 P
(43) Date of publication of application: 18.09.2024
(73) Proprietor: Alphamicron, Inc., Kent, OH 44240 (US)
(72) Inventor: TAHERI, Bahman, Shaker Heights, Ohio 44122 (US)
(74) Representative: Forresters IP LLP
(86) International application number: PCT/US2022/049468
(87) International publication number: WO 2023/086426

(56) References cited:
- EP-A1- 3 185 068
- WO-A1-2020/097295
- US-A- 5 032 007
- US-A- 5 347 378
- US-A1- 2006 038 929
- US-A1- 2019 091 069

## Description

### CROSS-REFERENCE TO RELATED APPLICATIONS

This application claims priority to, and any other benefit of, U.S. Provisional Patent Application Serial No. 63/263,923 entitled LIGHT PROTECTION SYSTEM AND METHOD, filed November 11, 2021.

### TECHNICAL FIELD

Embodiments of the subject matter disclosed herein relate to a system and method of using optical polarization techniques to attenuate light from a light source.

### BACKGROUND

In many situations, it is important to control impinging undesirable light to prevent harm or damage to, for example, a user's eyes, devices, areas, sensitive equipment, among others or to allow better visibility or contrast for systems such as augmented or mixed realities. Eye damage due to laser light or spark flash may be of great concern. For example, laser light can cause eye damage to pilots or first responders, and spark flash can cause eye injury to electrical grid works.

To accommodate these concerns, electronically switchable optical filters (active optical filters) may be used to attenuate light in various applications to adjust/reduce the level of light passing through the device. By way of example and not limitation, attenuating light filters can be used in goggles, glasses, windshields, and windows, among others.

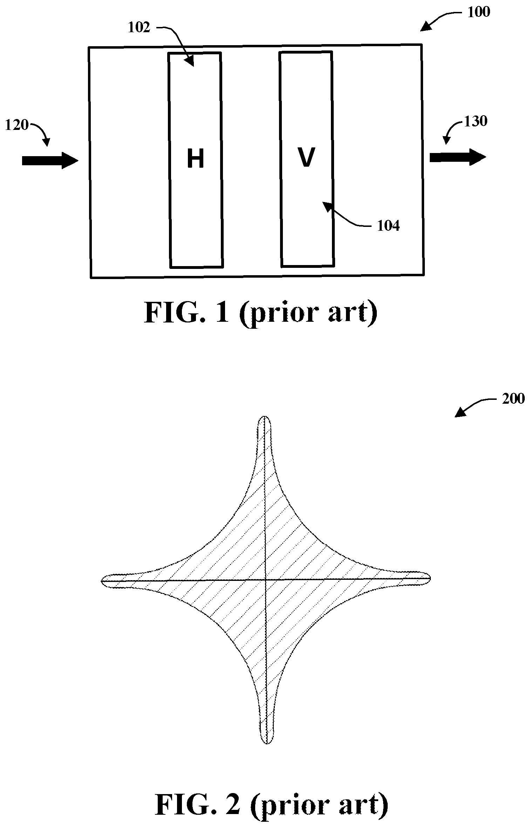

Various devices use optical filters to attenuate and adjust levels of light passing through them. One method of light attenuation is through an optical filter formed from a pair of polarizers that sandwich a conventional liquid crystal waveplate that acts as an electronically controllable polarization rotator. This geometry is commonly used in conventional LCDs. In these filters the maximum optical attenuation level is dictated by the performance of the polarizers. As an example of such a device, FIG. 1 illustrates an optical filter **100** formed from a first static (not electronically switchable) absorptive polarizer **102** and a second static absorptive polarizer **104** that acts on light **120** from a light source to produce transmitted light **130** which may be attenuated in intensity. The first static absorptive polarizer **102** is configured to polarize light on a first axis (horizontal) and the static absorptive second polarizer **104** is configured to polarize light on a second axis orthogonal to the first axis (vertical), thereby forming a pair of crossed polarizers. Crossed polarizers are highly effective at eliminating light incident normal to its surface (i.e., normally incident light). However, they do not operate as effectively when light is incident at oblique angles. Therefore, the attenuated intensity from a wide viewing angle has a cross pattern. FIG. 2 illustrates a typical cross pattern **200** of attenuated light formed by a pair of broadband cross polarizers of FIG. 1. This is an inherent limitation of conventional polarizer-based devices and results in light that is attenuated less effectively at the outer "corners". The light leakage at the outer corners results in an inhomogeneous attenuation of light that limits the optical performance of the filter. The role of the liquid crystal wave plate in these devices is to control the polarization state of the light that has exited the first polarizer without absorptive losses. Its presence, however, results in decreased field of effective attenuation (FEA). As such, a significant amount of effort is placed in finding ways to mitigate this negative effect, including using compensated polarizers, etc.

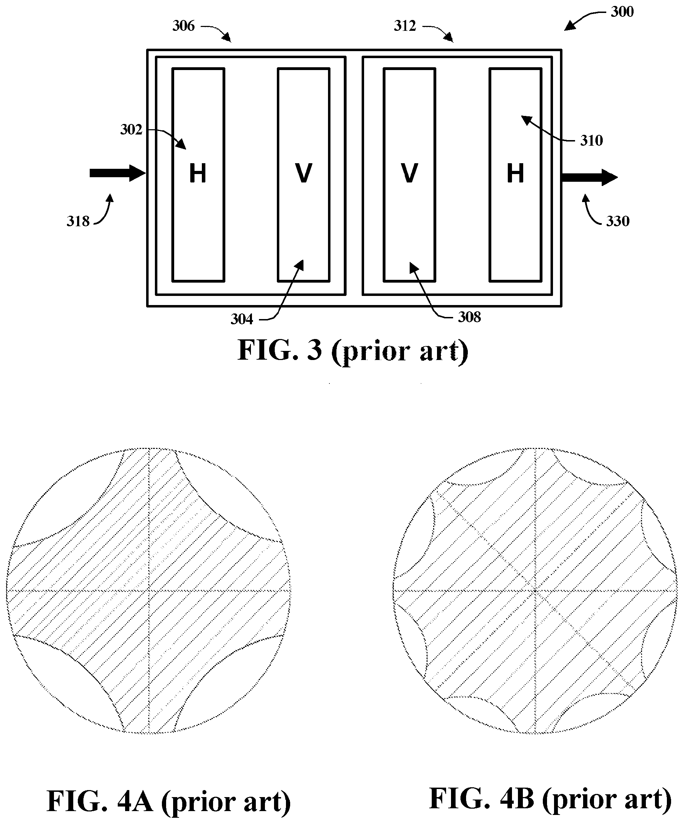

For example, FIG. 3 illustrates an approach to address this reduced FEA. Optical filter **300** includes two horizontal polarizers and two vertical polarizers, where the two vertical polarizers are in the center. In other words, the optical filter **300** is formed from two sets of crossed static absorptive polarizers, namely a first polarizer pair **306** and a second polarizer pair **312** that acts on light emerging from **306** to produce transmitted light **330,** which may be attenuated in intensity. The first and second polarizer pair **306, 312** each include a horizontal polarizer **302, 308** and a vertical polarizer **304, 310,** respectively. Fig. 4A shows the pattern of light attenuation formed by the prior art optical filter **300** of FIG. 3.

The FEA can be further improved by rotating the second set of cross polarizers with respect the first. Fig. 4B shows the pattern when the second cross polarizer pair **312** is rotated at a 45-degree angle to the first pair **306.** This configuration can improve device inhomogeneity of attenuated light but suffers from other limitations, i.e., reduced clear state transmission, which is undesired in certain applications.

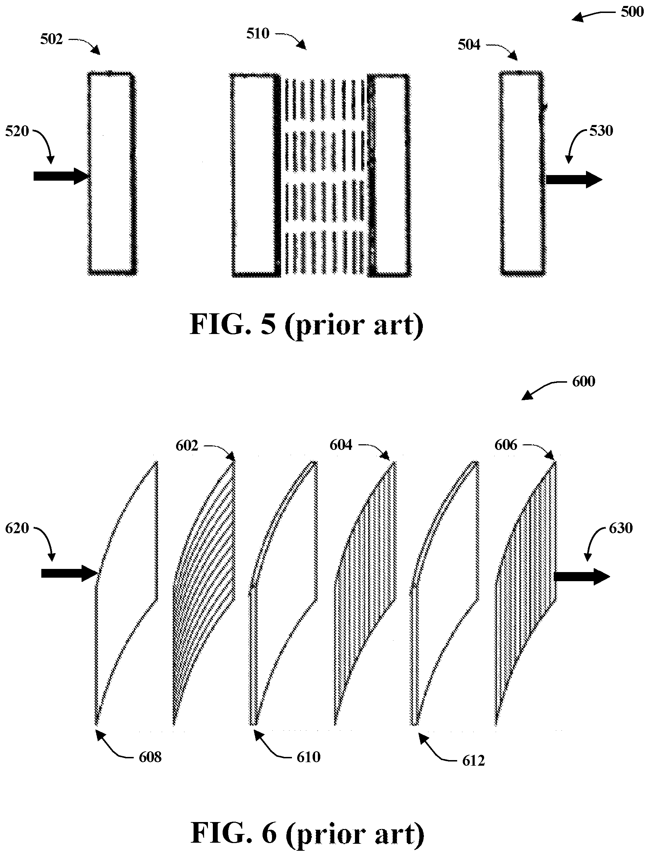

In the optical filter examples above, a sandwiched liquid crystal cell can be used to alter the polarization of light emerging from the first static absorptive polarizer and thus alter light transmission through the second static absorptive polarizer. For example, FIG. 5 illustrates a prior art example using an active liquid crystal waveplate (or polarization rotator) in optical filter **500** that includes a first static absorptive polarizer **502,** a second static absorptive polarizer **504,** and a conventional liquid crystal cell **510** located between the first and the second polarizers **502** and **504.** The polarizers **502** and **504** are plane polarizers where the first polarizer **502** is rotated 90 degrees from the second polarizer **504.** Optical filter **500** acts on light **520** from a light source

to produce transmitted light **530.** The conventional liquid crystal cell **510** is a non-absorbing, electrically controllable phase retarder or waveplate, that can rotate the polarization of light by 90 degrees. By alternating the voltage applied to the waveplate **510,** optical filter **500** can alternate between a clear and a dark state.

Similarly, two or more active liquid crystal waveplates can be used in more complex configurations. FIG. 6 illustrates a prior art optical filter **600** that includes a plurality of liquid crystal layers **(608, 610, 612)** and a plurality of static absorptive polarizers **(602, 604, 606).** The polarizer **602** is rotated ninety-degrees from the direction of the polarizers **604, 606,** which are parallel to each other. Optical filter **600** acts on light **620** to produce transmitted light **630,** which may be attenuated in intensity across a broad spectrum. When activated, the active liquid crystal cells can rotate the polarization of received light to achieve a desired darkness. For wide-band applications, this arrangement is complex and not ideal for near-eye applications due to its low clear state transmission.

For near eye applications, the need for a wide visible angle plays a significant role in the applicability of the device. This has hindered the implementation of electronically variable shutters in a variety of eyewear form factors. Therefore, it is desirable to provide simple optical filters that have good visible angle dependence for near-eye applications.

EP 3 185 068 discloses a method of making a curved switchable filter. US 2019/091069 discloses protective headgear with a curved switchable shutter. US 5 032 007 discloses an electronic color filter system for controlling a picture element color signal. US 5 347 378 discloses a frame-sequential color video system and color-selective filters for use in the same. WO 2020/097295 discloses an optical assembly.

### SUMMARY

An optical filter for attenuating a light source is provided. The optical filter includes first and second outer polarizers each configured to effectively polarize light in a first polarization axis. A first inner switchable polarizer is disposed between the first outer polarizer and the second outer polarizer. The first inner switchable polarizer is configured to alternate between a more polarizing state and a less polarizing state upon a change of an applied voltage. The first inner switchable polarizer configured to polarize light on a second polarization axis effectively orthogonal to the first polarization axis when the first inner switchable polarizer is in the more polarizing state. The change in applied voltage alters a transparency of the optical filter between a more transparent state and a less transparent state. The switchable polarizer may include a liquid crystal cell. The optical filter may include multiple switchable polarizers.

These and other objects of this invention will be evident when viewed in light of the drawings, detailed description, and appended claims.

### BRIEF DESCRIPTION OF THE DRAWINGS

Reference is made to the accompanying drawings in which particular embodiments and further benefits of the provided subject matter are illustrated as described in more detail in the description below.
FIG. 1 shows a prior art optical filter formed from a pair of crossed static absorptive polarizers.
FIG. 2 shows a typical pattern of light attenuation by the optical filter of FIG. 1.
FIG. 3 shows a prior art optical filter formed from two horizontal polarizers and two vertical polarizers, where the two vertical polarizers are in the center.
FIG. 4A shows a typical pattern of light attenuation by the optical filter of FIG. 3.
FIG. 4B shows a typical pattern of light attenuation by the optical filter of FIG. 3 where the second pair of polarizers are rotated at a 45-degree angle from the first pair.
FIG. 5 shows a prior art optical filter formed from a pair of static absorptive polarizers and a single non-absorbing polarization rotator.
FIG. 6 shows a prior art optical filter formed from multiple static absorptive polarizers and multiple non-absorbing polarization rotators.
FIG. 7A is a non-limiting depiction of a Field of Effective Attenuation (FEA).
FIG. 7B is a non-limiting aperture that may be acceptable for an optical filter.
FIG. 7C is another non-limiting depiction of a FEA for a human.
FIG. 8 is a schematic of a non-limiting example of an optical filter system according to some embodiments.
FIG. 9 is a cross-sectional schematic view of a non-limiting example of an optical filter system in a transmissive state according to some embodiments.
FIG. 10 is a cross-sectional schematic view of a non-limiting example of an optical filter system in a darkened state according to some embodiments.
FIG. 11 is a cross-sectional schematic view of a non-limiting example of a guest host liquid cell switchable polarizer according to some embodiments.
FIG. 12 is a cross-sectional schematic view of a non-limiting example of an optical filter system according to some embodiments.
FIG. 13 is a representation of the absorbance of light when a red filter of the optical filter of FIG. 12 is activated.
FIG. 14 is a representation of the absorbance of light when a red filter, a blue filter, and a green filter of the optical filter of FIG. 12 are activated.
FIG. 15 is a cross-sectional schematic view of a non-limiting example of an optical filter system according to some embodiments.

### DETAILED DESCRIPTION

### DEFINITIONS

Unless specifically defined otherwise herein, the definitions for optical parameters such as linear, circular, and unpolarized light are the same as those in "Principles of Optics Electromagnetic Theory of Propagation, Interference and Diffraction of Light", Max Born, et al., Cambridge University Press; 7th edition (Oct. 13, 1999). Similarly, all liquid crystal terminology which is not specifically defined herein is to have the definition as used in Liquid Crystals Applications and Uses, vol. 3, edited by B. Bahadur, published by World Scientific Publishing Co. Pte. Ltd., 1992 ("Bahadur").

**"Acceptance Aperture"** refers to the total angle where the optical density is above an accepted value.

**"Active"** as used herein means electronically switchable.

**"Band Pass Filter"** as used herein, is defined as a device that allows light of a certain range of wavelength(s) to pass through but rejects the passage of light of other wavelengths.

**"Clear"** or **"Clear State"** means a maximally transmissive state. In some embodiments, a clear state has a light transmission greater than 10%, 15%, 20%, 25%, 30%, 35%, 40%, 45%, 50%, 55%, 60%, 65%, 70%, 75%, 85%, or 90%.

**"Contrast Ratio"** as used herein, is defined as a ratio of the transmitted light in fully transmissive state to the transmitted light in the fully dark state. In some embodiments, the contrast ratio is greater than 2, 5, 10, 25, 50, 75, or 100.

**"Dark"** or **"Dark State"** means a minimally transmissive state. In some embodiments, a dark state has a light transmission of 25%, 10%, 5%, 1%, 0.5%, 0.1%, 0.01%, 0.001%, 0.0001% or less.

**"Effective Polarization"** or **"Effective Polarization Axis"** refers to the polarization or polarization axis produced by a polarizer (linear, circular, or other) either alone or in combination with an adjacent waveplate if one is present.

**"Field of Effective Attenuation" or FEA** is a solid angle beyond which the optical density drops by a preset value. The preset value can be greater than 0.1, 0.2, 0.3, 0.5, 1, 1.5 or 2 ODs.

**"Narrow-Band"** region of the spectrum is a region having a full-width, half-max of less than 175 nm.

**"Optical device"** is any device that can control the transmission of light. For example, a viewer or sensor can see through the device.

"Optical filter" as used herein, is a device that selectively transmits light of different wavelengths or different wavelength regions. A "wavelength region" refers to a band within the electromagnetic spectrum with a range of wavelengths, the band may be narrow (<175 nm) or wide (≤175 nm).

**"Orthogonal Axis"** refers to the orientation of a circular or linear polarizer element axis with respect to an incident polarized light such that a least transmission of the circularly or linearly polarized light respectively is achieved.

**"Optical Density" or "OD":** OD is related to the absorbance (A) and is defined as: $OD = - log A$

Transmissivity, T, is then related to the OD as: $T = {10}^{- OD}$

**"Static"** means a non-switchable or passive layer.

**"Polarizer"** refers to a material that absorbs or reflects one polarization of incident light more than the orthogonal polarization.

**"Polarization Axis"** refers to the direction along which a polarizer passes the electric field of an electromagnetic wave. For example, an absorptive or reflective polarizer having an X polarization axis will preferentially transmit the x-direction polarization and absorb or reflect a portion of the y-direction polarization of incident light, etc.

**"Switchable Polarizer"** refers to an optical layer that can be switched or altered, by application of voltage change, to alternate between a more polarizing state (absorbing or reflective) and a less polarizing (more transmissive) state. In other words, in the more polarizing state, the polarizer preferentially absorbs or reflects one polarization of light at a greater rate than in the less-polarizing state. In a less polarizing state, the polarizer will effectively transmit both polarization axes of light. The polarization level of the switchable polarizer is selected by controlling the voltage applied. Depending on the application, a switchable polarizer can be in a maximal polarizing state when a max voltage is applied, or vice versa, (i.e., be maximally polarizing when the voltage is 0).

**"Tinted"** state as used herein, is defined as a state between the maximally transmissive (clear) and the minimally transmissive (dark) states.

**"Transparent"** is used interchangeably with Clear.

**"Wide-band"** or **"Broad-band"** region of the spectrum is a region having a full-width, half-max equal to or greater than 175 nm.

As used in this application, the terms "x" and "y" polarization axes are arbitrary and refer to a first and second polarization axis of light which are orthogonal axes to each other and can be linear, circular, or elliptical. For example, they may be used to describe a first and second polarization axis. This is used to simplify and/or aid in the disclosure of the present application and is not to be construed as any fixed values or axis. Furthermore, terms such as parallel and perpendicular may be used to describe two orthogonal polarization states, which can be circular, elliptical, or linear, for example. In some cases, a first polarization axis may refer to a right-handed circularly polarized light, and an orthogonal second polarization axis may refer to the opposite left-handed circularly polarized light (or *vice versa* with respect to right- and left-handedness). These terms, parallel and perpendicular, can also be used in the same sense to describe the response of the polarizers to the incident polarization state.

Unless otherwise noted, the polarization created by a polarizer generally refers to its "effective" polarization in the context of the optical device and adjacent optical layers such as waveplates (as a non-limiting example). Similarly, an axis of polarization that is described as parallel means "effectively parallel" in the context of the optical device, and an axis of polarization that is described as orthogonal means "effectively orthogonal" in the context of the optical device and adjacent optical layers. Although the term "effective" may not always be used out of brevity, it is generally implied unless otherwise specifically noted or inconsistent with the discussion. For example, it is well known that the polarization state of the light entering or exiting a polarizer can be altered with appropriate use of waveplates such as a half-wave plate, quarter-wave plate etc. In such a case the polarizer is creating an "effective" polarization. This effective polarization can be linear or circular and can have an axis which matches or is different from the axis of the polarizer. For example, transmitted light from a circular polarizer can be turned into a linear polarization by use of a proper quarter-wave plate. Conversely, transmitted light from a linear polarizer can be turned into a circular polarization by use of an appropriate quarter-wave plate placed at the appropriate angle with respect to the polarizer axis. This allows for other polarizer systems or configurations to be used. For example, a pair of perpendicularly oriented static polarizers can be utilized as outside polarizers in conjunction with appropriate static wave-plates instead of two parallel-oriented polarizers in the examples described below. Similarly, a combination of circular and linear polarizers with appropriate wave-plates can be used instead of two reflective or two linear polarizers to match the operation of a switchable polarizer. For example, an absorptive switchable polarizer can be used in conjunction with two circular polarizers if additional wave plates are used.

With reference to the drawings, like reference numerals designate identical or corresponding parts throughout the several views. However, the inclusion of like elements in different views does not mean a given embodiment necessarily includes such elements or that all embodiments of the invention include such elements.

The systems and methods described herein provide optical filters that can increase peripheral performance, response time, or the contrast ratio of the overall device. For example, by utilizing three polarizers in a configuration such that the two outer polarizers are static absorptive or reflective polarizers with a polarization axis effectively parallel to each other, and the middle or inner polarizer is an electronically switchable polarizer configured to be orthogonal to the other two. In such a configuration, the discernable Field of Effective Attenuation or FEA can be controlled or modified. Therefore, this arrangement may be used to enhance the angular dependence of a polarization-based shutter and result in an increased FEA.

In an exemplary embodiment, an optical filter can include a first static absorptive or reflective polarizer, a second static absorptive or reflective polarizer having a polarization axis parallel to the polarization of the first polarizer, and a switchable polarizer provided between the first polarizer and the second polarizer. The first and second polarizers may sometimes be referred to herein as outer polarizers and the switchable polarizer in the middle may be referred to as an inner switchable polarizer. The switchable polarizer may include a switchable liquid crystal (LC) cell having a polarization direction that is alterable by an application of (or removal of) voltage to the LC cell, i.e., applying a voltage change. Therefore, by applying a voltage change to the LC cell, the transparency of the optical filter can be adjusted between a clear state, a dark state, or any state therebetween, where the optical filter absorbs or reflects more light in the dark state than in the clear state. In some embodiments, the electronically switchable polarizer utilizes an LC cell configured to reflect light within a broad-band or a narrow-band region of the electromagnetic spectrum. In some examples, the electronically switchable polarizer utilizes a guest host LC configuration. In some embodiments, the first and/or second polarizers may instead be electronically switchable outer polarizers having a polarization axis substantially orthogonal to that of the inner switchable polarizer.

It should be noted that switchable polarizer LC cells of the present disclosure are different than conventional LC cells of the prior art discussed in the background. As mentioned, conventional LC cells such as those of FIG. 6 include waveplates designed to rotate the polarization of received light without reducing the transmitted energy. In other words, they are ideally designed to transmit all of the incident light but rotate its polarization. Conversely, switchable polarizer LC filters of the present disclosure are configured to reduce (e.g., by absorption or reflection) the incident light transmitted energy.

In another embodiment, an optical filter is configured to selectively reduce narrow-band regions of the spectrum (for example for laser eye-protection or similar applications). This configuration can include a first outer polarizer, a second outer polarizer having a polarization direction the same as the first polarizer (in parallel), and two or more electronically switchable polarizers (inner polarizers) located between the first and the second outer polarizers. For example, the two or more switchable polarizers can include liquid crystal cells configured to polarize in a direction effectively orthogonal to the first and the second outer polarizers. The inner switchable polarizers can be individually controlled and configured such that each, or at least one, operates (polarizes) a narrow-band region of the spectrum. The optical filter can then selectively transmit or block a narrow-band region of the spectrum as needed. This feature can have applications to block intense light such as from a laser without blocking other regions of the spectrum.

In some embodiments, the optical filter may include three switchable polarizers. The first switchable polarizer (first liquid crystal cell) can be placed between the first outer polarizer and the second switchable polarizer (second liquid crystal cell). The third switchable polarizer (liquid crystal cell can be placed between the second switchable polarizer (second liquid crystal cell) and the second outer polarizer. For instance, the order of components can be: the first outer polarizer, the first switchable polarizer, the second switchable polarizer, the third switchable polarizer, and the second outer polarizer.

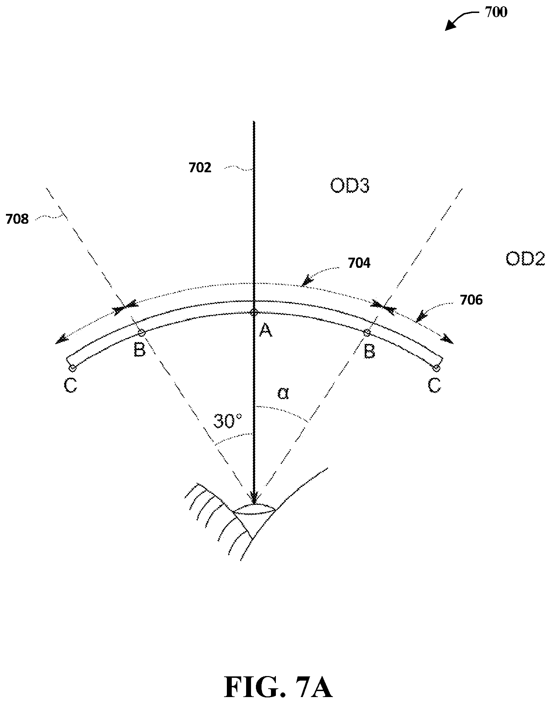

The arrangement of components described herein will affect the size and quality of the Field of Effective Attenuation or FEA. FIG. 7A illustrates an exemplary FEA 700 for a human being. In this example, the line 702 represents a normal direction of zero degrees. Angle α represents the angle from normal 702 that can define an acceptance aperture. The acceptance aperture can be related to angle α such that angle α represents the angle in which the optical density (OD) rating drops by a percentage, e.g., one (1) compared to the OD rating at normal 702. For example, a device that has an OD3 rating at normal 702 may have an OD2 rating at angle α. In an example, the acceptance aperture or FEA may be 30 degrees from normal 702, illustrated by the line 708. For instance, location 704 illustrated by line B-B represents a 60-degree FEA with an OD3 rating. The location 706 illustrated by line B-C represents a location having an OD2 rating. It should be appreciated that any angle may be used to define an acceptance aperture and that 30 degrees is provided solely for exemplary purposes. The FEA value may be 5°, 10°, 15°, 20°, 30°, 40°, 50°, 60°, 70°, 80°, 90°, 120° or higher.

Further, there may be a gradient in optical density and the dashed line **708** may simply indicate where one side is closer to OD = 3 and the other side is closer to OD = 2.

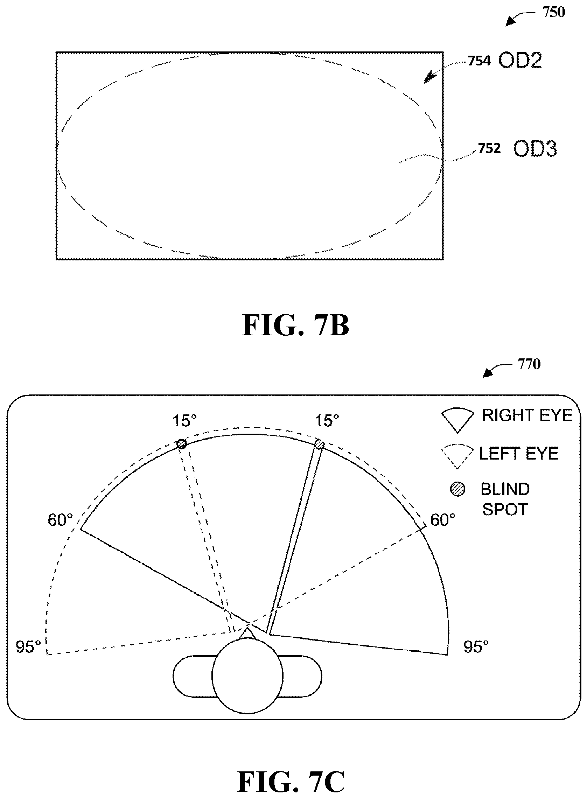

FIG. 7B illustrates an exemplary acceptance aperture **750** when looking through a polarizer. The acceptance aperture is defined in the middle portion **752** of the aperture which may have an OD3 rating. The corners or outer portions **754** can be considered outside of the acceptance aperture and may have a lower OD rating, e.g., OD2. In an example, having a large acceptance aperture **752** is desirable over a smaller acceptance aperture because a larger acceptance aperture can lead to a larger FEA. The size of the acceptance aperture can be controlled to mitigate undesirable light leakage at high angles as illustrated by cross pattern **200,** for example. A device that is more efficient at blocking out intense light sources can have a higher acceptance aperture and, therefore, may offer a wider FEA.

FIG. 7C illustrates an example of a field of view **770** for a human being, measured in degrees eccentric to fixation, in which the human eye can perceive images and detect motion. In certain applications, it is desirable to make the FEA such that it matches the field of view **770.** In this example, the field of view or FEA is about 155 degrees for each eye, or a total 190 degrees for both eyes together. In other examples, to achieve a desirable OD rating, a device can be configured to produce a reduced FEA by presenting a smaller viewing window to account for the configuration of the polarizers. Therefore, it may be desirable to produce an optical filter that can achieve an adequate OD rating while maintaining a wide FEA.

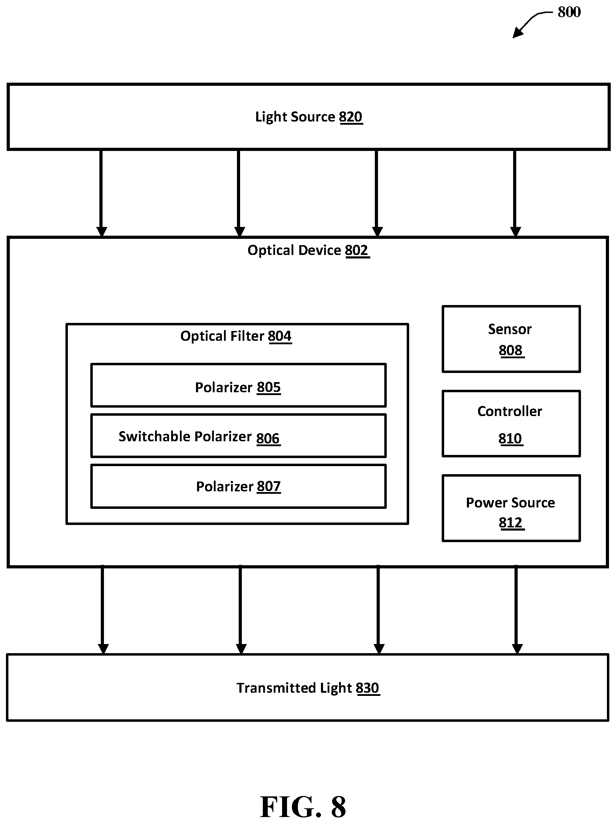

FIG. 8 illustrates a system **800** configured to attenuate light from a light source. The optical device **802** can include an optical filter **804,** a sensor **808,** a controller **810** including a processor and memory, and a power source **812.** The system **800** can include one optical device **802** or a plurality of optical devices. The optical device **802** can include one optical filter **804** or a plurality of optical filters. Similarly, sensor **808** may include a single sensor or represent a plurality of sensors. It should be appreciated that the system **800** can be configured with and can include all of or a portion of the components mentioned elsewhere herein. Similarly, the system **800** can optionally include additional components not mentioned herein without departing from the scope of the invention.

In some embodiments, the optical device **802** can include one or more sensors **808** for sensing an amount of light energy from the light source **820.** In an example, the sensor **808** can be chosen from any of the following types: photoresistors, photodiodes, phototransistors, and the like.

The controller **810** can include one or more processor(s) configured to include computer-executable instructions such as instructions such as a control process for an optical filter device. Such computer-executable instructions can be stored on one or more computer-readable media including non-transitory, computer-readable storage media such as memory. For instance, memory can include non-volatile storage to persistently store instructions, settings, configuration settings, parameter settings, light intensity setpoints, and/or data. Memory can also include volatile storage that stores instructions, other data (working data or variables), or portions thereof during execution by processor.

The power source **812** can include a battery of any of the following technologies: lithium ion, lithium polymer, nickel cadmium, nickel-metal hydride, lead acid, or any other suitable composition. In other examples, the power source **812** can be a shared power source for any other electrical device. Still, in other examples, the power source **812** can be a solar energy source. Power source **812** may include a power grid or a generator and may be DC or AC. It should be appreciated that the power source **812** may include a combination of any of the above-mentioned sources of power.

The optical filter **804** can include first and second polarizers, **805** and **807** having a first polarization axis, and a switchable polarizer **806** characterized by a second polarization axis, substantially orthogonal to the first axis. One or both of the outer polarizers **805, 807** may in some cases be static (non-switchable) polarizers. In some embodiments, one or both of outer polarizers **805** and **807** may be switchable or active so long as they have the requisite polarization axis. Polarizers **805, 806,** and **807** may be characterized as absorptive polarizers, reflective polarizers, broadband polarizers, or narrow band polarizers, among others.

An absorptive polarizer is a polarizer that can primarily absorb a selected polarization of light more than the other. An absorptive linear polarizer can have two propagation Eigen modes referred to herein as axes. These axes can include an absorptive axis and a transmissive axis, which can be orthogonal to each other. The polarization of the light that is aligned with the absorptive axis can be absorbed more than the perpendicular polarization. In some examples, the polarizer can be designed/configured to be wavelength dependent. In some cases, a broadband polarizer may polarize a broad band of light, e.g., light having a full-width, half-max absorption equal to or greater than 175 nm. In some cases, a narrowband polarizer may polarize a narrow band of light, e.g., light having a full-width, half-max absorption of less than 175 nm.

For example, an absorptive linear polarizer with an axis in the x-direction can mean that the polarizer will substantially absorb the x-direction polarization of light while substantially allowing y-direction polarization to propagate. Consequently, an absorptive linear polarizer with an axis in the y-direction means that the polarizer can substantially absorb y-polarized light and substantially transmit x-polarized light. In the examples depicted in the figures, X and Y can be used for the first and second polarization directions (e.g., Eigen modes) for ease of explanation, but it is to be understood that it may represent any polarization state. For example, the polarization states can be any of the following: circular, elliptical, linear, or the like. In another example, absorptive circular polarizers may be constructed by using a linear polarizer in combination with a quarter wave retarder. Once light is polarized by the polarizer, the quarter wave plate can induce a 1/4 phase retardation that can turn a linear polarization to a circular polarization.

A reflective polarizer is a polarizer that may reflect a selected polarization of light more than the other. For example, a reflective polarizer with a reflective axis in the x-direction can indicate that the reflective polarizer will reflect a circular polarization (for example right-handed) of incident light more than the other y-direction polarization (left-handed). Consequently, a reflective polarizer with a reflective axis in the y-direction means that the reflective polarizer can reflect the x-direction polarization (right) of incident light more than the other y-direction polarization (left). As in the case of absorptive polarizers, X and Y can be used to indicate the first and second polarization states for ease of explanation. It should be appreciated, however, that any polarization states such as circular, elliptical, or linear can be used. In the case of circular light, the x and y refer to the handedness of the polarization rather than fixed directions in space. Therefore x, for example, will denote right circular and y will denote left circular. As in the case of linearly polarized light, the unpolarized light will be considered to be composed of equal amounts of left and right circularly polarized amounts. The reflective polarizer, in that case, will reflect either the right- or the left-circular polarization depending on its configuration and will transmit the other polarization, left or right, respectively.

In some cases, a broadband reflective polarizer may polarize a broad band of light, e.g., having a full-width, half-max reflection of 175 nm or greater. In some cases, a narrowband reflective polarizer may polarize a narrow band of light, e.g., having a full-width, half-max reflection of less than 175 nm.

In some embodiments, a polarizer (absorptive, reflective, or other) may be in the form of a single layer structure, or alternatively, have a multilayer structure.

In an example, light can enter optical device **802** as light **820** and exit as light **830.** Light **830** can be attenuated by optical device **802** by a certain percentage. For instance, the optical device **802** can be configured to achieve a dark state which is minimally transmissive, a clear or transparent state which is maximally transmissive, or a tinted state falling between the dark state and the transparent state, by manipulating the voltage applied to the switchable polarizer .**806.** That is, the transparency of the optical filter **804** may be altered between a more transparent state (which may optionally include a clear state in some cases) and a less transparent state (which may optionally include a dark state in some cases).

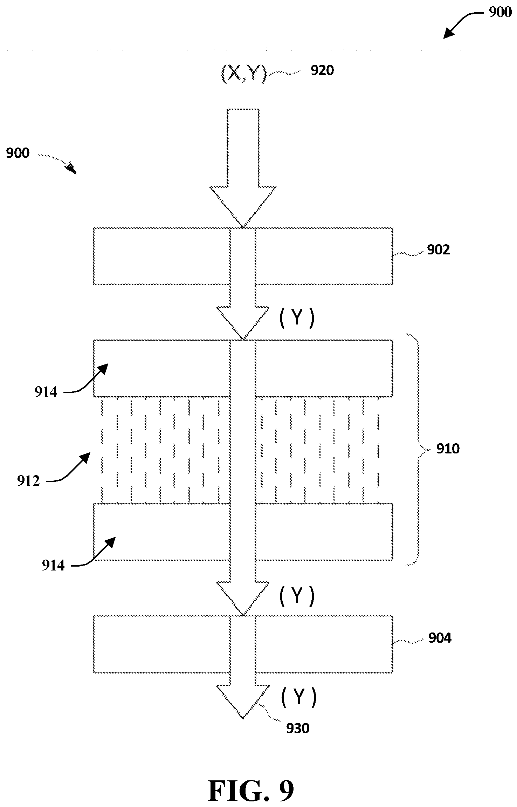

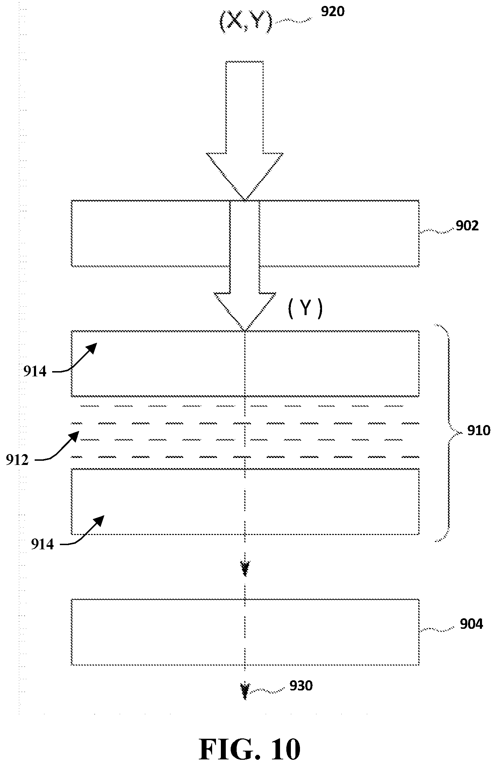

FIGS. 9 and 10 illustrates an exemplary optical filter **900.** In some embodiments, the optical filter **900** may provide an increased viewing angle or FEA with minimal compromise in desired light attenuation or response time when activated. The optical filter **900** includes a first polarizer **902** and second polarizer **904,** where the first and the second polarizers are considered outer polarizers. The optical filter **900** can further include an inner switchable polarizer **910** provided between the first polarizer **902** and the second polarizer **904.** The first polarizer **902** and the second polarizer **904** can each have a first polarization axis effectively parallel to one another, and the inner switchable polarizer **910** can have a second polarization axis effectively orthogonal to that of the first polarizer **902** and the second polarizer **904.** In some embodiments, the first and the second polarizers **902** and **904** can be static (non-switchable) polarizers. In some other embodiments, one or both of the first and second polarizers **902** and **904** can be switchable polarizers that have or can produce a polarization axis effectively parallel to each other and approximately orthogonal to the inner switchable polarizer in some instances as needed.

In some embodiments, whether switchable or not, a second polarizer **904** may have its physical axis of polarization positioned at an angle that is substantially perpendicular to the polarization axis of the first polarizer **902,** provided that there is a waveplate or polarization rotator positioned between the first polarizer and the second polarizer (e.g., between the second polarizer **904** and the inner switchable polarizer **910).** The waveplate can rotate received polarized light by 90 degrees to make the second polarizer effectively parallel to the first polarizer.

In some cases, the inner switchable polarizer **910** may include a guest host liquid crystal cell. The inner switchable polarizer **910** may be switched between a more polarizing state and a less polarizing state.

In some embodiments as shown in FIGS. 9 and 10, the first polarizer **902** and the second polarizer **904** can each have a first polarization axis that is transmissive to Y-polarized light, but is generally not transmissive to X-axis polarized light. Inner switchable polarizer **910** is switchable between a less polarizing state and a more polarizing state. The less polarizing state may be generally transmissive to both polarizations of light (X and Y) such that incident light may pass through switchable polarizer **910** without substantial attenuation. The more polarizing state may be generally transmissive to X-polarized light, but generally not transmissive to Y-polarized light.

FIG. 9 illustrates optical filter **900** in a more transparent or clear state. In operation, non-polarized light **920** having both X and Y polarizations (X,Y) impinges on first polarizer **902** having a first polarization axis that transmits Y-polarized light, but that generally does not transmit X-polarized light. The first polarizer may be absorptive or reflective as previously discussed. Thus, predominantly Y-polarized light reaches the inner switchable filter **910** which is in a less polarizing or transmissive state. In this example, the inner polarizer is a guest-host LC cell having a positive anisotropy dichroic dye guest in a negative dielectric anisotropy LC. In this configuration, a less polarizing state means the liquid crystal and dye of the guest host cell **912** align in a direction approximately perpendicular to the cell substrates **914,** referred to as homeotropic alignment. The incident Y-polarized light is thus transmitted through the inner switchable polarizer **910** without substantial attenuation (when in this less polarizing state). The Y-polarized light is then incident on the second polarizer, which is also generally transmissive to the Y-polarized light, to produce transmitted light **930.** It should be noted that some X-polarized light may also be present, but in this case, the majority of the light **930** is Y-polarized. In some embodiments, the transmissivity in the more transmissive state can be at least about 10%, alternatively at least about 12%, 15%, 18%, 20%, 25%, 30%, 40% or 50%.

In some applications, it is desirable to achieve a device transmissivity that is higher than 50%. One way this can be achieved is to use switchable polarizers for one or both outer polarizers **902** and **904.** For example, if polarizer **902** is a switchable polarizer in its less polarizing (more transparent) state, then both X- and Y-polarizations of incident light **920** will be transmitted through with relatively low attenuation to inner switchable polarizer **910.** When inner switchable polarizer **910** is in its less polarizing (more transparent) state it will pass the incident X-and Y-polarized light through to outer switchable polarizer **904** with relatively low attenuation. If outer switchable polarizer **904** is in its less polarizing (more transparent) state, it will pass transmitted light **930** with relatively low attenuation. Thus, when all switchable polarizers **902, 910** and **904** are in their less polarizing state, the optical filter may in some cases have a transmissivity of at least 50%, 60%, 70%, 80%, or 90%.

FIG. 10 illustrates the optical filter **900** in a less transmissive state, e.g., in a dark state. Here, the inner switchable polarizer **910** is in its polarizing state designed to preferentially absorb or reflect Y-polarized light. In this example, the switchable polarizer **910** is in a planar or homogeneous alignment where the liquid crystal and dye are aligned in a direction that is approximately parallel to the substrates **914.** As such, a portion of the incident Y-polarized light is blocked (absorbed or reflected). As a result, light emerging from optical filter **910** as transmitted light **930** is substantially attenuated. Note that even in a dark state, there can be some small amount of light transmitted. In some embodiments, e.g., where the guest host cell is chosen to have a broad band absorption, the optical filter **900** can achieve a transmissivity of about 1% corresponding to an OD 2 rating. In some embodiments, the transmissivity can be 0.1% corresponding to an OD 3 rating, 0.01% corresponding to an OD 4 rating, or 0.001% corresponding to an OD 5 rating. In some embodiments, the dark state OD may be at least 1.0, alternatively at least 1.5, 2.0, 2.5, 3.0, 3.5, 4.0, 4.5 or 5.0.

The inner switchable polarizer **910** can be switched by a change in an applied voltage. For example, inner switchable polarizer **910** may be in a more transmissive or clear state (less polarizing) when no voltage is applied, and in a less transmissive or dark state (more polarizing) when a voltage is applied. Alternatively, the inner switchable polarizer **910** may be in a less transmissive or dark state (more polarizing) when no voltage is applied, and in a clear state (less polarizing) when a voltage is applied.

In some embodiments, when the optical filter is in its less transparent state, transmitted light **930,** to the degree that it is visible, may appear neutral color (almost black or gray). For example, this may be the case when using a switchable polarizer that reflects or absorbs broad-band light across the visible spectrum. In some embodiments, transmitted light **930** may have a distinct color. For example, this may be the case when using a switchable polarizer that reflects or absorbs narrow-band light within the visible spectrum.

In some embodiments, the inner switchable polarizer can be a cholesteric LC, known as a CLC. Reflective CLCs are known in the art.

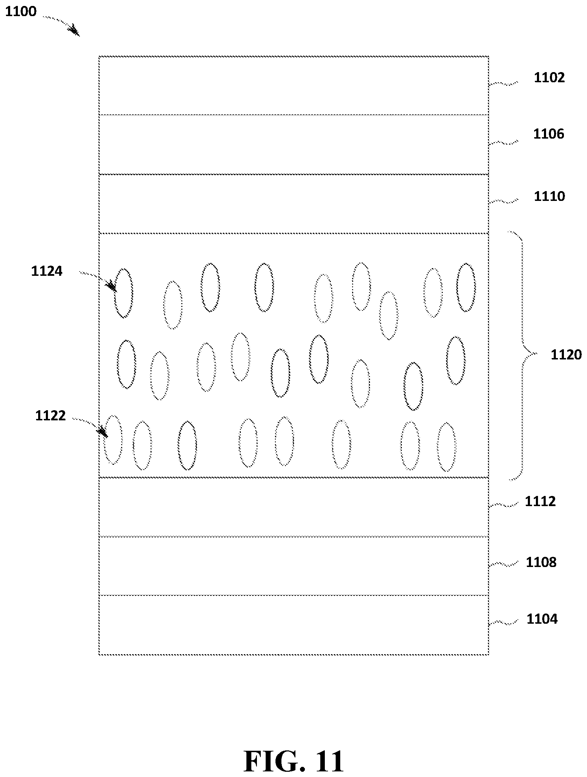

FIG. 11 illustrates an exemplary guest host (GH) liquid crystal (LC) cell **1100.** The guest host liquid crystal cell **1100** can include substrates **1102** and **1104,** electrode layers **1106** and **1108,** and a guest host liquid crystal mixture **1120** disposed between the substrates. The guest host liquid crystal mixture can include a plurality of liquid crystals **1122** and/or dye **1124** which may be a dichroic dye. The transparent electrode layers **1106** and **1108** can be disposed on the substrates **1102** and **1104** such that each electrode is interposed between its respective substrate and the guest host liquid crystal mixture. Substrates **1102** and **1104** may be flexible or rigid and may be made from a plastic or a glass material. Some non-limiting examples of substrates include polycarbonate and Corning Willow Glass. Substrates are generally transparent, but in some cases, may have a hue or coatings that affect the optical transmission.

In an exemplary embodiment, the liquid crystal cell **1100** may include a first alignment layer **1110** disposed on the electrode layer **1106** and a second alignment layer **1112** disposed on the electrode layer **1108.** In an example, spacers (not shown) can be used to maintain separation between various layers of the guest host liquid crystal cell **1100.** The electrode layers **1106** and **1108** can be indium tin oxide (ITO) or other suitable transparent conductive materials. In another embodiment, the liquid crystal cell **1100** can include first alignment layer **1110** and not second alignment layer **1112.** Yet, in another embodiment, the liquid crystal cell **1100** can include second alignment later **1112** and not first alignment layer **1110.**

In an embodiment, the liquid crystal **1122** can have a positive anisotropy or, in alternative embodiments, can have a negative anisotropy. Similarly, the dichroic dye **1124** can be chosen to have a positive, or in alternative embodiments, a negative dielectric anisotropy. In an example, the liquid crystal cell gap can be between 3-50 microns. In other examples, the cell gap can be around 4-12 microns with a spatial variation of +/- 10%. The contrast ratio of the filter can be greater than 7:1, 10:1, 20:1, 50:1, 100:1, 1000:1, or 10,000:1.

In certain embodiments, the liquid crystal cell **1100** can be flat. Yet, in other embodiments, the liquid crystal cell **1100** can have curvature in at least one dimension and can have a diopter of greater than zero. In other embodiments, the liquid crystal cell **1100** can have curvature in multiple directions with curvature along one direction of at least 0.1, 0.5, 1, 2, 3, 4, 5, or 6, for example.

In some examples, the guest host mixture **1120** includes a host liquid crystal **1122** and at least one guest dye or dye mixture **1124.** In some cases, a switchable polarizer using such a guest host mixture can have a switchable polarization that can be altered between a more transmissive or less polarizing state, where both polarization directions of light are transmitted, and a more polarizing state, where one polarization direction of light is reflected or absorbed more than the other. For example, the inner switchable polarizer **910** can alternate between the more transmissive state and the less transmissive state by changing the voltage applied to the electrodes **1106** and **1108.** As mentioned, the electric field can cause the liquid crystal **1122** and dye **1124** molecules to orient themselves in a direction generally perpendicular to the orientation of the substrates **1102** and **1104** forming a homeotropic orientation or parallel to the substrates **1102** and **1104** forming a planar orientation.

By way of example, a homeotropic state while voltage at the liquid crystal cell **1100** is zero (e.g., V=0) can be achieved when there is -ve LC at the liquid crystal cell (e.g., clear at rest or at V=0). A planar state at V=0 can be achieved when there is +ve LC at the liquid crystal cell (e.g., dark at rest or V=0). When using a positive dye, the guest host mixture can absorb light when in a planar orientation and can transmit light when in a homeotropic orientation. A polarizer state can be described as when liquid crystal **1122** and dye **1124** align in a generally parallel orientation to the substrates **1102** and **1104.** A transmissive state can be described as when liquid crystal **1122** and dye **1124** align in a generally perpendicular orientation to the substrates **1102** and **1104** allowing transmission of light. The change in applied voltage can be done automatically, manually, or remotely through a control board (not shown). The voltage can also be controlled by a controller, such as controller **810,** for example, when a certain level of light at one or more sensors **808** is detected. In some examples, the guest-host mixture can have an order parameter that is greater than 0.6, or 0.7, 0.71, 0.72, 0.73, 0.74, 0.75, 0.76, 0.77, 0.78, 0.79 or 0.8, or any number in between 0.6 and 0.8.

In an embodiment, the optical filter **900** can be configured for wide band absorption. In some applications, the entire visible light spectrum or a wide band may need to be absorbed. Wide band absorption can be defined as a spectral absorption band that is greater than 175 nm in one embodiment, and greater than 180 nm, 185 nm, 190 nm, 195 nm or 200 nm in other embodiments, where the entire spectral absorption band is contained within the range of visible wavelengths, typically assumed to be 400 nm-700 nm.

In an example, wide band absorption can be accomplished by using a wide band dye. The absorption band width, or the width of the absorption curve, may be calculated by what is known in the art as an "Aggregate Full Width at Half Maximum" (A-FWHM). In this example, a wide band dye can be considered to be any dye or mixture of dyes that results in a spectral absorption band characterized by an A-FWHM that is equal to or greater than 175 nm in one embodiment, and greater than 180 nm, 185 nm, 190 nm, 195 nm or 200 nm in other embodiments, where the entire spectral absorption band is contained within the range of visible wavelengths. A-FWHM can be understood as follows: full width at half maximum (FWHM) is a measurement that characterizes the width of the absorption curve of the dye. It is defined as the distance between the cut-off points on an absorption curve that occur where the absorption is one-half of the maximum absorption. Thus, regardless of the shape of the absorption curve, the FWHM is the width of the curve between the two cut-off points where the absorption is one-half of the maximum absorption.

In some embodiments, a polarizer or optical filter can be configured for narrow band absorption. In certain applications, it is desirable to have a switchable inner polarizer (e.g. a guest host cell) that can block certain wavelengths, or wavelength regions, of light according to what is needed. For example, if laser light of a particular color or wavelength is directed towards the optical filter, it may be desirable for the optical filter to adapt quickly and block the selected region of the spectrum that corresponds to the laser. This can reduce or eliminate damage caused by the laser light. In other embodiments, the optical filter can be adapted to have two or more guest host cells, where each can be designed to block a particular region of the light spectrum. In these embodiments, the device can be capable of switching between the two guest host cells as the incident laser or other bright light source changes color or wavelength.

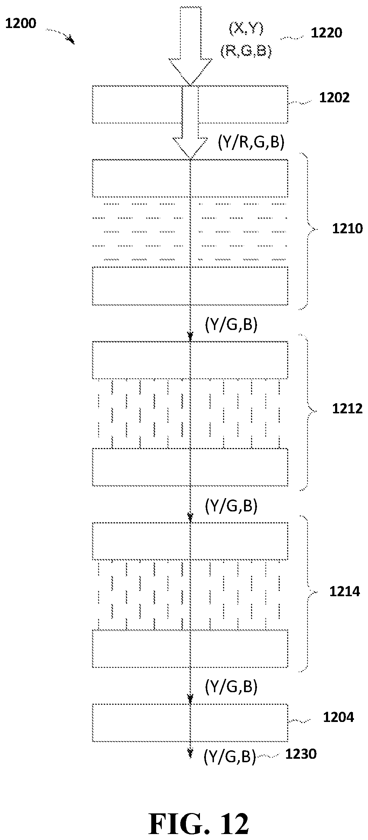

FIG. 12 illustrates an exemplary embodiment of an optical filter **1200.** The optical filter **1200** can include a first polarizer **1202** and a second polarizer **1204,** which may also be referred to as the outer polarizers. The optical filter **1200** can further include a first switchable polarizer **1210,** a second switchable polarizer **1212,** and a third switchable polarizer **1214,** referred to as the switchable polarizers or inner switchable polarizers.

In this embodiment, first, second, and third switchable polarizers **1210, 1212,** and **1214** can include guest host liquid crystal cells. Each guest host liquid crystal cell can be configured to filter a particular region of the light spectrum. The first and second polarizers **1202** and **1204,** respectively, can have an effectively parallel polarization axis thereby absorbing or reflecting the same polarization axis of light. In some embodiments, the optical filter **1200** can also include a band pass filter (not shown) to filter out desired wavelengths such as infrared and ultraviolet light. In other embodiments, the optical filter **1200** can be curved in shape (not shown).

In one example, when guest host cells of the switchable polarizers are in their clear or transparent state, the liquid crystal and dye can be in a homeotropic orientation to allow light **1220** to pass through un-polarized. In a dark state, the liquid crystal and dye can be in a planar orientation, and can absorb or reflect a specified region of light in the spectrum.

The operation may in some cases be generally analogous to that described with respect to optical filter **900.** First and second outer polarizers may each be characterized by a first polarization axis, i.e., they are effectively parallel to each other in this regard. The inner switchable polarizers (in the more polarizing state) are characterized by a second polarization axis that is effectively orthogonal to the first polarization axis.

For example, the first polarizer **1202** and the second polarizer **1204** can each have a first polarization axis that is transmissive to Y-polarized light, but is generally not transmissive to X-axis polarized light. Inner switchable polarizers **1210, 1212, 1214** are individually switchable between a less polarizing state and a more polarizing state. A less polarizing state may be generally transmissive to both polarizations of light (X and Y) such that incident light may pass through a switchable polarizer without substantial attenuation. A more polarizing state may be generally transmissive to X-polarized light, but generally not transmissive to Y-polarized light. Although first and second polarizers **1202** and **1204** are discussed in this example as static polarizers, in some embodiments one or both can also be switchable polarizers.

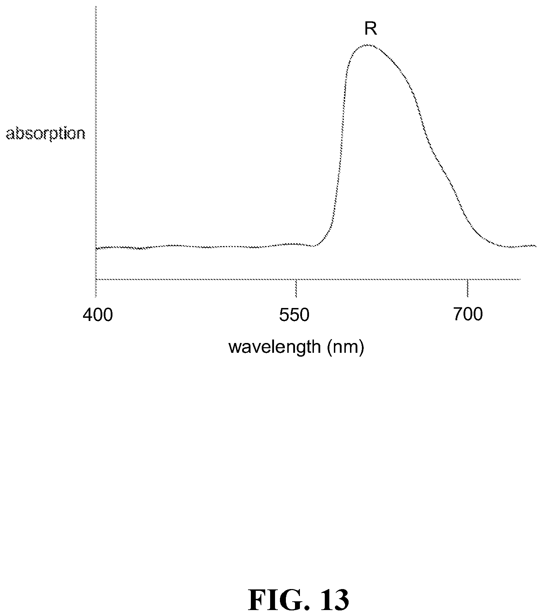

In operation, non-polarized white light having red (R), green (G), and blue (B) color components and both X and Y polarizations (X,Y) may impinge the outer first polarizer **1202** which allows only Y-polarized red, green and blue light to pass (Y/R,G,B). In FIG. 12, the first switchable red polarizer **1210** is in its more polarizing state so that it absorbs or reflects the incident red light, but allows the green and blue light through. The other switchable polarizers are each in their less polarizing state and also allow the Y-polarized green and blue light through. Outer second polarizer **1204** similarly allows Y-polarized green and blue light to be transmitted so the optical filter **1200** in this configuration transmits light **1230** that is cyan (blue-green) in color, with a substantial amount of the red light from light **1220** now removed. A representative absorption spectrum of the optical filter **1200** in this configuration is shown in FIG. 13.

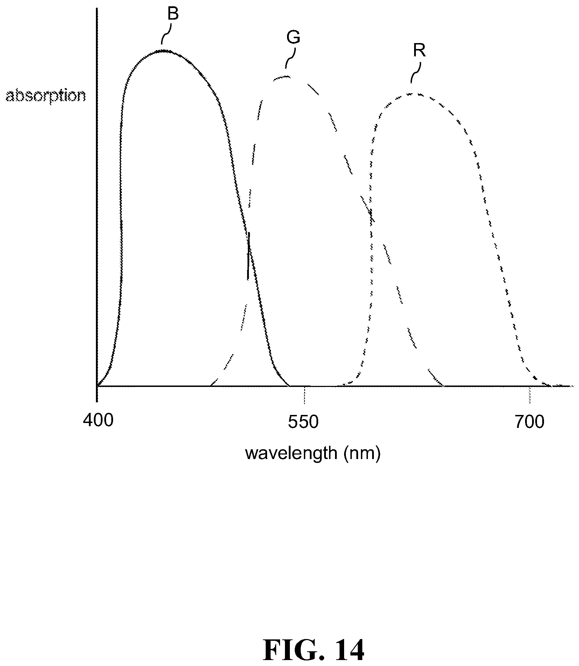

In some other embodiments, instead of the first switchable polarizer **1210** being in its more polarizing state, just the green second switchable polarizer **1212** may be, or alternatively just the blue third switchable polarizer **1214** may be in a polarizing state. Representative absorption spectra for these states are shown in FIG. 14 where spectrum R is that for the red first switchable polarizer in its more polarizing state, spectrum G is that for the green second switchable polarizer in its more polarizing state, and spectrum B is that for the blue third switchable polarizer in its more polarizing state. Any combination of red, green, and blue switchable filters may be in their more polarizing state. Further, as indicated elsewhere the intensity of light attenuation for each color can in some cases be controlled across a range rather than simply switched between two states.

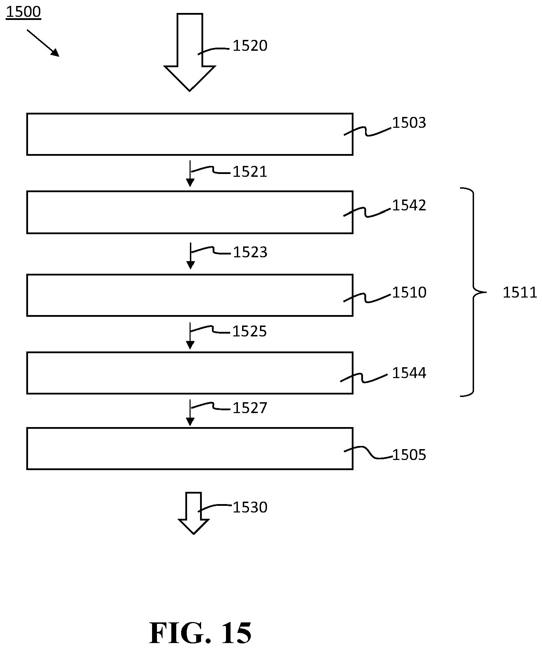

FIG. 15 illustrates another non-limiting example of an optical filter according to some embodiments. Optical filter **1500** may include a first cholesteric liquid crystal (CLC) polarizer **1503,** a second CLC polarizer **1505,** an absorptive switchable polarizer **1510** disposed between the first and second CLC polarizers, a first quarter wave plate **1542** disposed between the first CLC polarizer and the absorptive switchable polarizer, and a second quarter wave plate **1544** disposed between the absorptive switchable polarizer and the second CLC polarizer. In some cases, the switchable polarizer **1510** and quarter wave plates **1542, 1544** may collectively be considered a switchable multilayer absorptive circular polarizer **1511.** In other non-limiting examples, the switchable polarizer can also alter the polarization state of the transmitted light in one of its states. This may allow for a normally dark or a normally clear device operation.

In some embodiments, the first and second CLC polarizers may be configured to reflect right-handed circularly polarized light. When the non-polarized light **1520** impinges on the first CLC polarizer **1503,** right-handed circularly polarized light is reflected, and left-handed circularly polarized light **1521** is transmitted through to the first quarter wave plate **1542.** The first quarter wave plate receives and acts on the left-handed circularly polarized light to produce substantially linearly polarized light **1523** having a first polarization axis.

In some cases, the LC cell may have a guest host configuration wherein the host may have a negative anisotropy. In its less polarizing state, switchable polarizer **1510** allows incident polarized light **1523** to pass substantially unaltered as polarized light **1525** (generally still polarized along a first polarization axis). In its more polarizing state, switchable polarizer may absorb or reflect a substantial amount of incident polarized light **1523** and allow relatively little light through as **1525.**

Polarized light **1525** (if any) is received by the second quarter wave plate **1544** which substantially transforms the polarized light having a first polarization axis to left-handed circularly polarized light **1527.** The second CLC polarizer **1505** receives and transmits the left-handed circularly polarized light to produce transmitted light **1530.** Note that right-handed circularly polarized light that may have leaked through or produced by the second quarter wave plate will be reflected by the second CLC polarizer. Although the first and second CLC polarizers were discussed as reflecting right-handed circularly polarized light, they both may instead be selected to reflect left-handed circularly polarized light.

It should be noted that switchable polarizer **1510** may in some embodiments be a reflective polarizer such as a CLC, rather than an absorptive, guest-host LC type of polarizer as described above. In such embodiments using a switchable reflective polarizer, the quarter wave plates may not be needed.

Still further embodiments herein include the following enumerated embodiments.
1. An optical filter comprising:
   first and second outer polarizers each configured to effectively polarize light in a first polarization axis; and
   a first inner switchable polarizer disposed between the first outer polarizer and the second outer polarizer,
   wherein the first inner switchable polarizer is configured to alternate between a more polarizing state and a less polarizing state upon a change of an applied voltage, the first inner switchable polarizer configured to polarize light in a second polarization axis effectively orthogonal to the first polarization axis when the first inner switchable polarizer is in the more polarizing state, and
   wherein the change in applied voltage alters a transparency of the optical filter from a more transparent state to a less transparent state.
2. The optical filter of embodiment 1, wherein the first inner switchable polarizer is an absorptive polarizer, a reflective polarizer, or a combination thereof.
3. The optical filter of embodiment 1 or 2, wherein the first inner switchable polarizer comprises a liquid crystal cell configured to absorb light comprising a mixture of a liquid crystal host and one or more guest dyes provided between a pair of substrates.
4. The optical filter of embodiment 3, wherein the one or more guest dyes includes an absorption band of 150 nm or greater.
5. The optical filter according to any of embodiments 3 - 4, wherein at least one of the pair of substrates comprises a flexible material.
6. The optical filter according to any of embodiments 1 - 5, wherein the less transparent state has an optical density (OD) of at least 2 for normally incident light.
7. The optical filter according to any of embodiments 1 - 6, further characterized by an acceptance aperture angle of at least 30 degrees from normally incident light, wherein an optical density rating at the acceptance aperture angle is decreased by no more than one as compared to an optical density rating of normally incident light.
8. The optical filter according to any of embodiments 1 - 7, wherein the optical filter is chosen from the following options:
   (a) configured to be in the more transparent state when the voltage is zero; or
   (b) configured to be in the less transparent state when the voltage is zero.
9. The optical filter according to any of embodiments 1 - 8, wherein the optical filter is flexible.
10. The optical filter according to any of embodiments 1 - 9, wherein the optical filter is curved.
11. The optical filter according to any of embodiments 1 - 10, wherein the first inner switchable polarizer is configured to absorb or reflect a narrow-band region of visible light or a broad-band region of visible light.
12. The optical filter according to any of embodiments 1 - 11, further comprising an infrared filter, an ultra-violet filter, or both.
13. The optical filter according to any of embodiments 1 - 14, further comprising a second inner switchable polarizer disposed between the first inner switchable polarizer and the second outer polarizer, wherein:
   i) the second inner switchable polarizer is configured to alternate between a more polarizing state and a less polarizing state upon a change of an applied voltage, the second inner switchable polarizer configured to polarize light in the second polarization axis when the second inner switchable polarizer is in its more polarizing state;
   ii) the first inner switchable polarizer is configured in its more polarized state to attenuate a first range of wavelengths of visible light; and
   iii) the second inner switchable polarizer is configured in its more polarized state to attenuate a second range of wavelengths of visible light.
14. The optical filter of embodiment 13, wherein the first range is different from the second range.
15. The optical filter of embodiment 13 or 14, wherein one or both of the first and second inner polarizers are configured to absorb or reflect a narrow-band region of visible light.
16. The optical filter according to any of embodiments 13 - 15, wherein the first range or the second range_of wavelengths comprise a range within a red light spectrum, a green light spectrum, or a blue light spectrum.
17. The optical filter of embodiment 13, wherein one or both of the first and second inner polarizers are configured to absorb or reflect a broad-band region of visible light.
18. The optical filter according to any of embodiments 13 - 17, further comprising a third inner switchable polarizer disposed between the second inner switchable polarizer and the second outer polarizer, wherein:
   i) the third inner switchable polarizer is configured to alternate between a more polarizing state and a less polarizing state upon a change of an applied voltage, the third inner switchable polarizer configured to polarize light in the second polarization axis when the third inner switchable polarizer is in its more polarizing state; and
   ii) the third inner switchable polarizer is configured in its more polarized state to attenuate a third range of wavelengths of visible light.
19. The optical filter of embodiment 18, wherein the third range is different than the first or second ranges
20. The optical filter of embodiment 19, wherein the third range of wavelengths comprises a range within a red light spectrum, a green light spectrum, or a blue light spectrum.
21. The optical filter of embodiment 18 or 19, wherein a color of transmitted light may be changed by selectively altering the polarization states of the inner first switchable polarizer, the inner second switchable polarizer, the inner third switchable polarizer, or a combination thereof.
22. The optical filter according to any of embodiments 1 - 21, wherein one or both of the first and second outer polarizers are configured as switchable polarizers.
23. The optical filter according to embodiment 22, wherein the more transparent state comprises greater than 50% transmissivity when all switchable polarizers are in the less polarizing state.
24. The optical filter according to any of embodiments 1 - 23, further comprising at least one waveplate positioned between an outer polarizer and an inner switchable polarizer.
25. The optical filter of embodiment 24, wherein the waveplate is a quarter waveplate.
26. The optical filter of embodiment 24, wherein the waveplate is a half waveplate.
30. An optical device comprising an optical filter according to any of embodiments 1 - 26, the optical device comprising a near-eye display, goggles, eyewear, a visor, a welding helmet, a window, a see-through display, or a space suit visor.

In the specification and claims, reference will be made to a number of terms that have the following meanings. The singular forms "a", "an" and "the" include plural referents unless the context clearly dictates otherwise. Approximating language, as used herein throughout the specification and claims, may be applied to modify a quantitative representation that could permissibly vary without resulting in a change in the basic function to which it is related. Accordingly, a value modified by a term such as "about" is not to be limited to the precise value specified. In some instances, the approximating language may correspond to the precision of an instrument for measuring the value. Moreover, unless specifically stated otherwise, a use of the terms "first," "second," etc., do not denote an order or importance, but rather the terms "first," "second," etc., are used to distinguish one element from another.

As used herein, the terms "may" and "may be" indicate a possibility of an occurrence within a set of circumstances; a possession of a specified property, characteristic or function; and/or qualify another verb by expressing one or more of an ability, capability, or possibility associated with the qualified verb. Accordingly, usage of "may" and "may be" indicates that a modified term is apparently appropriate, capable, or suitable for an indicated capacity, function, or usage, while taking into account that in some circumstances the modified term may sometimes not be appropriate, capable, or suitable. For example, in some circumstances an event or capacity can be expected, while in other circumstances the event or capacity cannot occur - this distinction is captured by the terms "may" and "may be."

This written description uses examples to disclose the subject matter, including the best mode, and also to enable one of ordinary skill in the art to practice the invention, including making and using a devices or systems and performing incorporated methods. The patentable scope of the invention is defined by the claims, and may include other examples that occur to one of ordinary skill in the art. Such other examples are intended to be within the scope of the claims if they have structural elements that do not differentiate from the literal language of the claims.

## Claims

1. An optical filter (804) comprising:
first (805) and second (807) outer polarizers each configured to effectively polarize light in a first polarization axis; and
a first inner switchable polarizer (806) disposed between the first outer polarizer (805) and the second outer polarizer (807), wherein the first inner switchable polarizer (806) is configured to alternate between a more polarizing state and a less polarizing state upon a change of an applied voltage, the first inner switchable polarizer (806) configured to polarize light in a second polarization axis effectively orthogonal to the first polarization axis when the first inner switchable polarizer (806) is in the more polarizing state, and
wherein the change in applied voltage alters a transparency of the optical filter (804) from a more transparent state to a less transparent state.

2. The optical filter (804) of claim 1, wherein the first inner switchable polarizer (806) is an absorptive polarizer, a reflective polarizer, or a combination thereof.

3. The optical filter (804) of claim 1 or 2, wherein the first inner switchable polarizer (806) comprises a liquid crystal cell configured to absorb light comprising a mixture of a liquid crystal host and one or more guest dyes provided between a pair of substrates (914), optionally wherein the one or more guest dyes includes an absorption band of 175 nm or greater, and optionally wherein at least one of the pair of substrates comprises a flexible material.

4. The optical filter (804) according to any of claims 1-3, wherein the less transparent state has an optical density (OD) of at least 2 for normally incident light.

5. The optical filter (804) according to any of claims 1-4, further **characterized by** an acceptance aperture angle of at least 30 degrees from normally incident light, wherein an optical density rating at the acceptance aperture angle is decreased by no more than one as compared to an optical density rating of normally incident light.

6. The optical filter (804) according to any of claims 1-5, wherein the optical filter (804) is chosen from the following options:
(a) configured to be in the more transparent state when the voltage is zero; or
(b) configured to be in the less transparent state when the voltage is zero.

7. The optical filter (804) according to any of claims 1-6, wherein the optical filter (804) is flexible.

8. The optical filter (804) according to any of claims 1-7, further comprising an infrared filter, an ultra-violet filter, or both.

9. The optical filter (840, 1200) according to any of claims 1-8, further comprising a second inner switchable polarizer (1212) disposed between the first inner switchable polarizer (1210) and the second outer polarizer (1204), wherein:
i) the second inner switchable polarizer (1212) is configured to alternate between a more polarizing state and a less polarizing state upon a change of an applied voltage, the second inner switchable polarizer (1212) configured to polarize light in the second polarization axis when the second inner switchable polarizer (1212) is in its more polarizing state;
ii) the first inner switchable polarizer (1210) is configured in its more polarized state to attenuate a first range of wavelengths of light; and
iii) the second inner switchable polarizer (1212) is configured in its more polarized state to attenuate a second range of wavelengths of light, different from the first range.

10. The optical filter (1200) of claim 9, wherein one or both of the first (1210) and second (1212) inner polarizers are configured to absorb or reflect a narrow-band region of visible light.

11. The optical filter (1200) of claim 9 or 10, wherein the first range or the second range of wavelengths comprise a range within a red light spectrum, a green light spectrum, or a blue light spectrum.

12. The optical filter (1200) according to any of claims 9-11, further comprising a third inner switchable polarizer (1214) disposed between the second inner switchable polarizer (1212) and the second outer polarizer (1204), wherein:
i) the third inner switchable polarizer (1214) is configured to alternate between a more polarizing state and a less polarizing state upon a change of an applied voltage, the third inner switchable polarizer (1214) configured to polarize light in the second polarization axis when the third inner switchable polarizer (1214) is in its more polarizing state; and
ii) the third inner switchable polarizer (1214) is configured in its more polarized state to attenuate a third range of wavelengths of light, different from the first and second ranges.

13. The optical filter (1200) of claim 12, wherein the third range of wavelengths comprises a range within a red light spectrum, a green light spectrum, or a blue light spectrum.

14. The optical filter (804) according to any of claims 1-13, wherein one or both of the first (805) and second (807) outer polarizers are configured as switchable polarizers.

15. An optical device (802) comprising the optical filter (804) according to any of claims 1- 14, the optical device (802) comprising a near-eye display, goggles, eyewear, a visor, a welding helmet, a window, a see-through display, or a space suit visor.

## Patentansprüche

1. Optisches Filter (804), umfassend:
einen ersten (805) und einen zweiten (807) äußeren Polarisator, jeweils ausgebildet zum wirksamen Polarisieren von Licht in einer ersten Polarisationsachse; und
einen ersten innerer schaltbaren Polarisator (806), der zwischen dem ersten äußeren Polarisator (805) und dem zweiten äußeren Polarisator (807) angeordnet ist, wobei der erste innere schaltbare Polarisator (806) zum Wechseln zwischen einem mehr polarisierenden Zustand und einem weniger polarisierenden Zustand bei einer Änderung einer angelegten Spannung ausgebildet ist, wobei der erste innere schaltbare Polarisator (806) zum Polarisieren von Licht in einer zweiten Polarisationsachse wirksam orthogonal zur ersten Polarisationsachse, wenn sich der erste innere schaltbare Polarisator (806) im mehr polarisierenden Zustand befindet, ausgebildet ist, und
wobei die Änderung der angelegten Spannung eine Transparenz des optischen Filters (804) von einem mehr transparenteren Zustand in einen weniger transparenten Zustand ändert.

2. Optisches Filter (804) nach Anspruch 1, wobei der erste innere schaltbare Polarisator (806) ein absorbierender Polarisator, ein reflektierender Polarisator oder eine Kombination daraus ist.

3. Optisches Filter (804) nach Anspruch 1 oder 2, wobei der erste innere schaltbare Polarisator (806) eine Flüssigkristallzelle umfasst, die zum Absorbieren von Licht ausgebildet ist, das eine Mischung aus einem Flüssigkristallwirt und einem oder mehreren Gastfarbstoffen umfasst, die zwischen einem Paar von Substraten (914) angeordnet sind, wobei der eine oder die mehreren Gastfarbstoffe optional ein Absorptionsband von 175 nm oder mehr umfassen und wobei optional wenigstens eines des Paares von Substraten ein flexibles Material umfasst.

4. Optisches Filter (804) nach einem der Ansprüche 1 bis 3, wobei der weniger transparente Zustand bei normal einfallendem Licht eine optische Dichte (OD) von wenigstens 2 aufweist.

5. Optisches Filter (804) nach einem der Ansprüche 1 bis 4, ferner **gekennzeichnet durch** einen Akzeptanzöffnungswinkel von wenigstens 30 Grad gegenüber normal einfallendem Licht, wobei ein optischer Dichtewert bei diesem Akzeptanzöffnungswinkel im Vergleich zu einem optischen Dichtewert von normal einfallendem Licht um nicht mehr als eins verringert ist.

6. Optisches Filter (804) nach einem der Ansprüche 1 bis 5, wobei das optische Filter (804) aus den folgenden Optionen ausgewählt wird:
(a) so ausgebildet, dass es sich im mehr transparenten Zustand befindet, wenn die Spannung null ist; oder
(a) so ausgebildet, dass es sich im weniger transparenten Zustand befindet, wenn die Spannung null ist.

7. Optisches Filter (804) nach einem der Ansprüche 1 bis 6, wobei das optische Filter (804) flexibel ist.

8. Optisches Filter (804) nach einem der Ansprüche 1 bis 7, ferner umfassend ein Infrarotfilter, ein Ultraviolettfilter oder beides.

9. Optisches Filter (840, 1200) nach einem der Ansprüche 1 bis 8, ferner umfassend einen zweiten inneren schaltbaren Polarisator (1212), der zwischen dem ersten inneren schaltbaren Polarisator (1210) und dem zweiten äußeren Polarisator (1204) angeordnet ist, wobei:
i) der zweite innere schaltbare Polarisator (1212) zum Wechseln zwischen einem mehr polarisierenden Zustand und einem weniger polarisierenden Zustand bei einer Änderung einer angelegten Spannung ausgebildet ist und der zweite innere schaltbare Polarisator (1212) zum Polarisieren von Licht in der zweiten Polarisationsachse, wenn sich der zweite innere schaltbare Polarisator (1212) in seinem mehr polarisierenden Zustand befindet, ausgebildet ist;
ii) der erste innere schaltbare Polarisator (1210) in seinem mehr polarisierten Zustand zum Dämpfen eines ersten Bereichs von Lichtwellenlängen ausgebildet ist; und
iii) der zweite innere schaltbare Polarisator (1212) in seinem mehr polarisierten Zustand zum Dämpfen eines zweiten Bereichs von Lichtwellenlängen, der sich vom ersten Bereich unterscheidet, ausgebildet ist.

10. Optisches Filter (1200) nach Anspruch 9, wobei einer oder beide von erstem (1210) und zweitem (1212) inneren Polarisator zum Absorbieren oder Reflektieren eines Schmalbandbereichs von sichtbarem Licht ausgebildet sind.

11. Optisches Filter (1200) nach Anspruch 9 oder 10, wobei der erste Bereich oder der zweite Bereich von Wellenlängen einen Bereich in einem Rotlichtspektrum, einem Grünlichtspektrum oder einem Blaulichtspektrum umfasst.

12. Optisches Filter (1200) nach einem der Ansprüche 9 bis 11, ferner umfassend einen dritten inneren schaltbaren Polarisator (1214), der zwischen dem zweiten inneren schaltbaren Polarisator (1212) und dem zweiten äußeren Polarisator (1204) angeordnet ist, wobei:
i) der dritte innere schaltbare Polarisator (1214) zum Wechseln zwischen einem mehr polarisierenden Zustand und einem weniger polarisierenden Zustand bei einer Änderung einer angelegten Spannung ausgebildet ist und der dritte innere schaltbare Polarisator (1214) zum Polarisieren von Licht in der zweiten Polarisationsachse, wenn sich der dritte innere schaltbare Polarisator (1214) in seinem mehr polarisierenden Zustand befindet, ausgebildet ist; und
ii) der dritte innere schaltbare Polarisator (1214) in seinem mehr polarisierten Zustand zum Dämpfen eines dritten Bereichs von Lichtwellenlängen, der sich vom ersten und zweiten Bereich unterscheidet, ausgebildet ist.

13. Optisches Filter (1200) nach Anspruch 12, wobei der dritte Bereich von Wellenlängen einen Bereich in einem Rotlichtspektrum, einem Grünlichtspektrum oder einem Blaulichtspektrum umfasst.

14. Optisches Filter (804) nach einem der Ansprüche 1 bis 13, wobei einer oder beide von erstem (805) und zweitem (807) äußeren Polarisator als schaltbarer Polarisatoren ausgebildet sind.

15. Optische Vorrichtung (802), umfassend das optische Filter (804) nach einem der Ansprüche 1 bis 14, wobei die optische Vorrichtung (802) eine Near-Eye-Anzeige, eine Schutzbrille, eine Sehhilfe, ein Visier, einen Schweißhelm, ein Fenster, eine durchsichtige Anzeige oder ein Raumanzugvisier umfasst.

## Revendications

1. Filtre optique (804) comprenant :
un premier (805) et un deuxième (807) polariseurs externes configurés chacun pour polariser efficacement la lumière selon un premier axe de polarisation ; et
un premier polariseur interne commutable (806) disposé entre le premier polariseur externe (805) et le deuxième polariseur externe (807), dans lequel le premier polariseur interne commutable (806) est configuré pour alterner entre un état plus polarisant et un état moins polarisant lors d'une modification d'une tension appliquée, le premier polariseur interne commutable (806) étant configuré pour polariser la lumière selon un deuxième axe de polarisation effectivement orthogonal au premier axe de polarisation lorsque le premier polariseur interne commutable (806) se trouve dans l'état plus polarisant, et
dans lequel la modification de la tension appliquée fait passer une transparence du filtre optique (804) d'un état plus transparent à un état moins transparent.

2. Filtre optique (804) selon la revendication 1, dans lequel le premier polariseur interne commutable (806) est un polariseur absorbant, un polariseur réfléchissant ou une combinaison de ceux-ci.

3. Filtre optique (804) selon la revendication 1 ou 2, dans lequel le premier polariseur interne commutable (806) comprend une cellule à cristaux liquides configurée pour absorber la lumière, comprenant un mélange d'un cristal liquide hôte et d'un ou plusieurs colorants invités disposé entre une paire de substrats (914), facultativement dans lequel les un ou plusieurs colorants invités incluent une bande d'absorption de 175 nm ou plus, et facultativement dans lequel au moins l'un de la paire de substrats comprend un matériau flexible.

4. Filtre optique (804) selon l'une quelconque des revendications 1 à 3, dans lequel l'état moins transparent présente une densité optique (DO) d'au moins 2 pour une lumière à incidence normale.

5. Filtre optique (804) selon l'une quelconque des revendications 1 à 4, **caractérisé en outre par** un angle d'ouverture d'acceptance d'au moins 30 degrés par rapport à une lumière à incidence normale, dans lequel un indice de densité optique à l'angle d'ouverture d'acceptance est inférieur d'au plus un à un indice de densité optique pour une lumière à incidence normale.

6. Filtre optique (804) selon l'une quelconque des revendications 1 à 5, dans lequel le filtre optique (804) est choisi parmi les options suivantes :
(a) configuré pour se trouver dans l'état plus transparent lorsque la tension est nulle ; ou
(b) configuré pour se trouver dans l'état moins transparent lorsque la tension est nulle.

7. Filtre optique (804) selon l'une quelconque des revendications 1 à 6, dans lequel le filtre optique (804) est flexible.

8. Filtre optique (804) selon l'une quelconque des revendications 1 à 7, comprenant en outre un filtre infrarouge, un filtre ultraviolet, ou les deux.

9. Filtre optique (840, 1200) selon l'une quelconque des revendications 1 à 8, comprenant en outre un deuxième polariseur interne commutable (1212) disposé entre le premier polariseur interne commutable (1210) et le deuxième polariseur externe (1204), dans lequel :
i) le deuxième polariseur interne commutable (1212) est configuré pour alterner entre un état plus polarisant et un état moins polarisant lors d'une modification d'une tension appliquée, le deuxième polariseur interne commutable (1212) étant configuré pour polariser la lumière selon le deuxième axe de polarisation lorsque le deuxième polariseur interne commutable (1212) se trouve dans son état plus polarisant ;
ii) le premier polariseur interne commutable (1210) est configuré, dans son état plus polarisé, pour atténuer une première plage de longueurs d'onde de lumière ; et
iii) le deuxième polariseur interne commutable (1212) est configuré, dans son état plus polarisé, pour atténuer une deuxième plage de longueurs d'onde de lumière, différente de la première plage.

10. Filtre optique (1200) selon la revendication 9, dans lequel l'un ou les deux parmi le premier (1210) et le deuxième (1212) polariseurs internes sont configurés pour absorber ou réfléchir une région à bande étroite de la lumière visible.

11. Filtre optique (1200) selon la revendication 9 ou 10, dans lequel la première plage ou la deuxième plage de longueurs d'onde comprend une plage située dans un spectre de lumière rouge, un spectre de lumière verte ou un spectre de lumière bleue.

12. Filtre optique (1200) selon l'une quelconque des revendications 9 à 11, comprenant en outre un troisième polariseur interne commutable (1214) disposé entre le deuxième polariseur interne commutable (1212) et le deuxième polariseur externe (1204), dans lequel :
i) le troisième polariseur interne commutable (1214) est configuré pour alterner entre un état plus polarisant et un état moins polarisant lors d'une modification d'une tension appliquée, le troisième polariseur interne commutable (1214) étant configuré pour polariser la lumière selon le deuxième axe de polarisation lorsque le troisième polariseur interne commutable (1214) se trouve dans son état plus polarisant ; et
ii) le troisième polariseur interne commutable (1214) est configuré, dans son état plus polarisé, pour atténuer une troisième plage de longueurs d'onde de lumière, différente des première et deuxième plages.

13. Filtre optique (1200) selon la revendication 12, dans lequel la troisième plage de longueurs d'onde comprend une plage située dans un spectre de lumière rouge, un spectre de lumière verte ou un spectre de lumière bleue.

14. Filtre optique (804) selon l'une quelconque des revendications 1 à 13, dans lequel l'un ou les deux parmi le premier (805) et le deuxième (807) polariseurs externes sont configurés sous la forme de polariseurs commutables.

15. Dispositif optique (802) comprenant le filtre optique (804) selon l'une quelconque des revendications 1 à 14, le dispositif optique (802) comprenant un affichage proche de l'œil, des lunettes de protection, des lunettes, une visière, un casque de soudage, une fenêtre, un dispositif d'affichage transparent ou une visière de combinaison spatiale.
